# EUROPEAN PATENT SPECIFICATION

(11) **EP 3 188 768 B1**
(45) Date of publication and mention of the grant of the patent: **27.05.2020**
(21) Application number: 15838231.7
(22) Date of filing: 04.09.2015
(51) Int. Cl.: A61M 1/00, A61B 17/00, G01N 33/483, G01N 33/49

(54) **SYSTEM FOR DETERMINING COMPONENTS OF MATTER REMOVED FROM A LIVING BODY AND RELATED METHODS**
SYSTEM ZUR BESTIMMUNG DER KOMPONENTEN VON AUS EINEM LEBENDEN KÖRPER ENTFERNTEM MATERIAL UND ZUGEHÖRIGE VERFAHREN
SYSTÈME DE DÉTERMINATION DE COMPOSANTS DE MATIÈRE ÉLIMINÉE D'UN CORPS VIVANT ET PROCÉDÉS ASSOCIÉS

(30) Priority: 04.09.2014 US 201462045705 P; 04.08.2015 US 201562200687 P
(43) Date of publication of application: 12.07.2017
(73) Proprietor: Werd, LLC, Lexington, KY 40515 (US)
(72) Inventor: KIRN, David S., Lexington, KY 40515 (US); HISEL, Richard D., Nicholasville, KY 40356-8105 (US); WHITMAN, William, Lexington, KY 40515 (US)
(74) Representative: Hanna Moore + Curley
(86) International application number: PCT/US2015/048596
(87) International publication number: WO 2016/037086

(56) References cited:
- WO-A1-2014/042890
- US-A- 4 562 842
- US-A- 4 770 187
- US-A- 4 962 041
- US-A- 5 709 670
- US-A- 5 709 670
- US-A- 5 817 032
- US-A1- 2002 151 874
- US-A1- 2012 308 536
- US-A1- 2012 308 536
- US-A1- 2013 301 901
- US-A1- 2014 207 103

## Description

### TECHNICAL FIELD

This document relates generally to a system for component identification, and more specifically to a system for determining and quantifying components of matter removed from a living body.

### BACKGROUND

Matter obtained from a patient is generally collected in a container or vessel having a graduated scale used for determining a quantity of the matter. The containers are typically disposable along with the matter collected therein in order to prevent health care worker exposure to biohazardous content. The matter, including bodily fluids, is collected in a variety of different medical settings including, but not limited to, suction aspirates during surgery, drains placed in body cavities, paracentesis aspirates, pleurocentesis aspirates, and liposuction procedures.

Depending on the procedure, information relating to the collected matter beyond just overall quantity can be utilized. For example, monitoring an amount of fluid and/or blood loss in any procedure is important for patient safety as the amount of aspirate guides replacement needs to avoid hypovolemic shock. In a liposuction procedure, as another example, the product of the surgical process, or the matter removed, includes a fluid component and a fat cells component. A majority of the fluid component is a tumescent fluid which was injected prior to the start of the liposuction process and consists of saline and trace amounts of local anesthetic drugs. Additional contributors to the fluid component include, among other possibilities, oil from ruptured fat cells and certain, typically small, amounts of blood.

A typical liposuction procedure involves treatment of multiple discrete areas on the same patient. The components which make up the matter will vary not only from patient to patient but even from area to area within the same patient. Hence, one area may yield a higher percentage of fat cells than another area. Accordingly, accurate quantification of the liposuction aspirate volume, or the matter removed, is important to ensure symmetric treatment of the left and right sides.

Liposuction systems typically use the same or similar collection vessels or containers as those used in the variety of different medical settings noted above. Commonly used containers include graduated scales for determining aspirate quantity which are notoriously imprecise due in part to a reliance on graduations of up to 50 mL or more. Even within this accuracy limit, readings must be performed with a level line of sight to avoid additional error. If the container is not located on eye level, as is often the case, additional error is likely to be imposed. It necessarily follows that the current standard for determining a quantity of liposuction aspirate in a container is highly imprecise. Even more, the error(s) is magnified as the overall volume is tracked from one patient area to another.

In addition to overall quantitative issues, there are currently no available means for accurately determining components of the matter removed from the body (e.g., a relative percentage or quantity of fat cells contained in the liposuction aspirate). In other words, there is no way to determine what portion of the matter removed from the patient is a fat cells component compared to, for example, a fluid component. As noted above, knowing this additional information would be particularly helpful to allow the surgeon to generate a symmetric treatment of left and right treatment areas.

Even more, the discontinuous and highly variable flow of matter being removed from the patient in a liposuction procedures makes measuring and/or determining components parts of the matter utilizing flow rate measurements by optical and weight determinations equally unreliable. Air gaps and periods of free air flow are normal with liposuction procedures. Accordingly, a need exists for a system capable of quantifying the matter removed and determining the components of the matter regardless of whether a flow of matter being removed is continuous or variable.

In addition, the system should be capable of quantification of the determined components without relying on a color of fat cells. Differentiating fat cells from fluid relying on a detected yellow color is highly unreliable since fat color can vary dramatically from one patient to another patient, and even from one anatomic area to another within the same patient. Even more, fat cell size, the degree of fat cell disruption during the aspiration process, and the amount of dilution by the tumescent fluid can effect fat color.

Even more, utilizing fat cells removed during the liposuction process for grafting has dramatically increased in popularity over the last decade. Typical applications include restoration of facial contours, buttock enhancement, or breast contour change in which the fat cells are reinjected into these areas immediately following a liposuction process. The most common method of performing fat cell grafting includes harvesting fat cells using a liposuction.

The resultant aspirate, or matter, is collected in a sterile container, usually placed on the surgical field, which is then connected to another non-sterile container on the liposuction pump. The aspirate from the liposuction process consists of a mixture of fat cells, fluid (a dilute saline mixture used for the liposuction process), oil from ruptured fat cells, and typically small amounts of blood. However, only the fat cell component of the aspirate is desirable for transfer to the recipient area. Accordingly, the system should be capable of separating fat cells from the matter removed from the patient including fluid in the liposuction aspirate. Preferably, the separation of the fat cells from the fluid occurs in a rapid, atraumatic process.

US 5,709, 670 A describes a monitor to provide substantially real-time estimates of fluid absorption rate and tissue loss rate in a patient during a surgical procedure. The monitor estimates fluid source flow rates and waste fluid flow rates based on weight changes per unit time or direct reading fluid flow rate meters.

US 4, 562, 842 A describes an apparatus for continuously monitoring the loss of blood suffered by a patient in the course of a surgical operation. The apparatus provides a means for sucking the combination of blood and irrigating solution gathered about the incision, and for sequentially measuring the volumes and densities of small samplings of the solution-and-blood mixture being drawn through the conduit leading from the incision area to the suction device.

US 2013/301901 A relates to a system and method for estimating a quantity of a blood component in a fluid canister.

US 4, 770, 187 A describes an apparatus for surgical aspiration and monitoring of the aspirated fluids which includes a sealable aspiration bottle supported by an electronic weighing scale on a base stand. A pair of electrodes extend into the bottle and are connected to an electronic circuit which measures the impedance of the fluid/tissue aspirate in the bottle.

### SUMMARY OF THE INVENTION

Insofar as the term invention or embodiment is used in the following, or features are presented as being optional, this should be interpreted in such a way that the only protection sought is that of the invention claimed. In accordance with the purposes and benefits described herein, a system for determining components of matter removed from a living body or patient is provided. The system may be broadly described as including a container for receiving the matter removed from the living body via an inlet tube, a device for weighing said container and the matter received therein, an instrument for obtaining information relating to at least one aspect of the matter in said container, and a processor programmed to determine the components of the matter in said container based in part on a weight of the matter determined by said device and the information relating to the at least one aspect of the matter obtained by said instrument.

In one possible embodiment, the system further includes a display including a screen for displaying information provided by the processor, the information relating to the matter in the container.

In another possible embodiment, the system further includes a transmitter for transmitting information including at least the weight of the matter in the container and the information relating to at least one aspect of the matter in the container, and wherein the display includes a receiver for receiving the transmitted information.

In yet another possible embodiment, the system further includes a transmitter for transmitting information provided by the processor, the information relating to the matter in the container, and wherein the display includes a receiver for receiving the transmitted information.

In still another possible embodiment, the system further includes a mobile device including the display. In another, the mobile device is a smart phone, a personal digital assistant, or a laptop computer. In other possible embodiments, the screen is a touch screen.

In one other possible embodiment, the system further includes a memory associated with the processor for storing at least the weight of the matter determined by the device and the information relating to the at least one aspect of the matter obtained by the instrument. In another, the memory further stores information relating to an area of the living body from which the matter was removed.

In another possible embodiment, the system further includes a switch for indicating to the processor a transition from one area of the living body from which the matter was removed to another area of the living body from which additional matter is to be removed. In another, the switch may be a momentary switch. In still another, the switch may be a foot switch or a toggle switch integrated within the touch screen.

In even more possible embodiments, the system further includes a pump connected to the container via a vacuum tube, the pump creating a vacuum within the system for suctioning the matter from the living body. In other possible embodiments, the system may further include a cannula connected to the container via the inlet tube, the cannula partially inserted into the living body for suctioning the matter from the living body. In another possible embodiment, the switch is a push button switch mounted on the cannula.

In still other possible embodiments, the instrument obtains information relating to a color of the matter in the container. In another, the processor is programmed to determine an amount of a blood component of the matter based in part on the weight of the matter determined by the device and the color of the matter in the container.

In yet another possible embodiment, the instrument obtains information relating to a height of the matter in the container. In other possible embodiments, the instrument includes at least one camera positioned adjacent the container for obtaining at least one image of the matter in the container. In still another, the processor is programmed to analyze the at least one image to determine a height of the matter in the container.

In another possible embodiment, the system further includes a digital pattern recognition analysis system to analyze the at least one image to determine a height of the matter in the container.

In yet other possible embodiments, the instrument includes at least two cameras positioned adjacent the container for obtaining at least one image from each of the at least two cameras of the matter in the container. In another, the processor is programmed to analyze the at least one image from each of the at least two cameras to determine a height of the matter in the container.

In another possible embodiment, the system further includes a digital pattern recognition analysis system to analyze the at least one image from each of the at least two cameras to determine a height of the matter in the container.

In still other possible embodiments, the instrument includes at least one mirror. In others, the instrument includes at least one light.

In still other possible embodiments, the camera is positioned adjacent a side of the container. In another, at least a portion of the container is clear. In yet others, the container includes graduation marks on a side of the container.

In yet another possible embodiment, the camera is positioned adjacent a top of the container.

In another possible embodiment, the at least one camera obtains information relating to a color of the matter in the container. In other possible embodiments, the processor is programmed to determine an amount of a blood component of the matter based in part on the weight of the matter determined by the device and the color of the matter in the container.

In yet other possible embodiments, the instrument is positioned adjacent a side of the container. In these embodiments, at least a portion of the container may be clear. In others, the container includes graduation marks on a side of the container.

In still other possible embodiments, the instrument senses a level of the matter in the container. In yet another possible embodiment, the instrument non-invasively senses a level of the matter in the container.

In one other possible embodiment, the system further includes a base for supporting the container, the device, and the instrument. In another, the base supports the display.

In other possible embodiments, the system further includes a base for supporting the container, and a clamp extending from the base for securing the inlet tube and the vacuum tube. In another embodiment, the clamp secures the inlet tube and the vacuum tube in order to isolate the container from at least one force applied to at least one of the inlet tube and the vacuum tube.

In yet another possible embodiment, the device for weighing the container and the matter received therein is a load cell. In another, the load cell is attached to the container.

In still another possible embodiment, the container is cylindrical in shape.

In still other possible embodiments, the instrument includes at least one optical sensor positioned adjacent the container and at least one corresponding receiver. In another, the at least one optical sensor is positioned adjacent the container and at least one corresponding receiver for obtaining the color of the matter in the container. In still another, the at least one optical sensor utilizes a red beam and an infrared beam and a red receiver and an infrared receiver.

In other possible embodiments, the instrument includes at least one optical sensor positioned adjacent the container and at least one corresponding receiver for obtaining a height of the matter in the container.

In another possible embodiment, the system further includes a liner positioned within the container for receiving the matter removed from the living body via the inlet tube. In another, the liner is disposable.

In still other possible embodiments, the system further includes an insert positioned within the container and containing an absorbent material, wherein the matter includes a fat cells component and a fluid component, and the insert is porous allowing the fluid component to enter the insert for absorption by the absorbent material. In another possible embodiment, the absorbent material is a superabsorbent polymer and/or a hydrogel.

In still another possible embodiment, the system further includes an inner container positioned within the container for receiving the matter from the patient, the inner container supporting the insert.

In yet still another possible embodiment, the system further includes a lid for the container, the lid including an inlet port connected to the inlet tube and a vacuum port connected to the vacuum tube through which the vacuum is applied to the container for suctioning the matter from the living body.

In one other embodiment, the insert includes a base and a tower, and a base of the inner container supports the insert base. In another, the absorbent material is positioned within the insert tower. In yet another, the absorbent material is positioned within the insert base and the insert tower.

In another possible embodiment, the tower includes an inner cavity within which the absorbent material is positioned, and a plurality of fins extending from a central axis. In another, the plurality of fins includes a plurality of holes allowing the fluid component to pass into the inner cavity of the tower for absorption by the absorbent material. In yet another, the plurality of fins includes a plurality of holes covered by a mesh filter, a paper filter, and/or a one-way fabric allowing the fluid to pass into the inner cavity of the tower for absorption by the absorbent material.

In still other possible embodiments, the insert includes a plurality of holes allowing the fluid to pass into an inner cavity of the insert for absorption by the absorbent material. In yet another, the insert includes a plurality of holes covered by a mesh filter, a paper filter, and/or a one-way fabric allowing the fluid to pass into an inner cavity of the insert for absorption by the absorbent material.

In still other possible embodiments, the inner container and the insert are integrally molded.

In yet one other possible embodiment, the instrument obtains information relating to a color of the liquid component in the container.

In another possible embodiment, the processor is programmed to determine an amount of a blood component of the matter based in part on the weight of the matter determined by the device and the color of the matter in the container. In still other embodiments, the processor is programmed to determine whether the container includes the inner container. In another embodiment, the processor is programmed to determine whether the container includes the insert. In yet another embodiment, the processor determines whether the container includes the inner container based on a tare weight of the container. In one other possible embodiment, the processor determines whether the container includes the inner container based on an input from a switch associated with the container.

In still another possible embodiment, the system further includes an insert positioned within the container and containing an absorbent material, wherein the matter includes a fat cells component and a fluid component, and the insert is porous allowing the fluid component to enter the insert for absorption by the absorbent material, and wherein the instrument obtains information relating to a height of the fat cells component in the container.

In another possible embodiment, the instrument includes at least one camera positioned adjacent the container for obtaining at least one image of the fat cells component of the matter in the container. In yet another embodiment, the processor is programmed to analyze the at least one image of the fat cells component of the matter to determine the height of the fat cells component in the container.

In accordance with the purposes and benefits described herein, a method is provided of determining components of matter removed from a living body. The method may be broadly described as comprising the steps of: (a) directing the matter removed from the living body into a container; (b) weighing the matter and the container; (c) obtaining information relating to at least one aspect of the matter in the container; and (d) determining the components of the matter in the container based in part on the weight of the matter and the information relating to at least one aspect of the matter.

In one possible embodiment, the the step of directing the matter includes applying a vacuum to the container for drawing the matter removed from the living body into the container. In another possible embodiment, the step of obtaining information relating to at least one aspect of the matter in the container includes obtaining information relating to a color of the matter in the container.

In still another possible embodiment, the step of determining the components of the matter in the container includes determining an amount of a blood component of the matter based in part on the weight of the matter and the color of the matter in the container.

In yet another possible embodiment, the step of obtaining information relating to at least one aspect of the matter in the container includes obtaining information relating to a height of the matter in the container.

In another possible embodiment, the step of obtaining information relating to the height of the matter in the container includes the step of analyzing at least one image of the matter in the container.

In one other possible embodiment, the step of determining the components of the matter in the container is based in part on the weight of the matter and the height of the matter in the container.

In another possible embodiment, the step of obtaining information relating to the height of the matter in the container includes the step of sensing a level of the matter in the container. In another, the step of determining the components of the matter in the container is based in part on the weight of the matter and the level of the matter in the container.

In another possible embodiment, the method further includes the step of separating a fluid component from a fat cells component of the matter. In another, the step of separating the fluid component from the fat cells component of the matter includes the step of drawing the fluid component through a filter using an absorbent material.

In still another, the step of obtaining information relating to at least one aspect of the matter in the container includes obtaining information relating to a height of the fat cells component in the container.

In another possible embodiment, the step of obtaining information relating to the height of the fat cells component in the container includes the step of analyzing at least one image of the fat cells component in the container.

In yet still another possible embodiment, the step of determining the components of the matter in the container is based in part on the weight of the matter and the height of the fat cells component in said container.

In the following description, there are shown and described several preferred embodiments of the system for determining components of matter removed from a living body or patient and related methods. As it should be realized, the various systems and methods are capable of other, different embodiments and their several details are capable of modification in various, obvious aspects all without departing from the systems as set forth and described in the following claims. Accordingly, the drawings and descriptions should be regarded as illustrative in nature and not as restrictive.

### BRIEF DESCRIPTION OF THE DRAWING FIGURES

The accompanying drawing figures incorporated herein and forming a part of the specification, illustrate several aspects of the system for determining components of matter removed from a living body or patient and related methods, and together with the description serve to explain certain principles thereof. In the drawing figures:
Figure 1 is a perspective view of a system for determining components of matter removed from a living body;
Figure 2 is a perspective view of a base supporting a platform and load cell for weighing a container and the matter removed from the living body;
Figure 3 is a perspective view of the base and support platform supporting a container for receiving the matter removed from the living body;
Figure 4 is a schematic illustration of the system;
Figure 5 is a detailed view of a display screen for the user interface at the touch screen display;
Figure 6 is a perspective view of an outer container with the lid removed revealing an inner container and an insert for housing an absorbent material;
Figure 7 is a perspective view of the inner container showing a base of the insert extending to an inner diameter of the inner container;
Figure 8 is a perspective view of the insert showing a cavity for receiving the absorbent material and a plurality of holes for liquid passage; and
Figure 9 is a perspective view of the insert separated from a base of the insert.

Reference will now be made in detail to the present embodiments of the system for determining components of matter removed from a living body or patient and related methods, examples of which are illustrated in the accompanying drawing figures, wherein like numerals are used to represent like elements.

### DETAILED DESCRIPTION

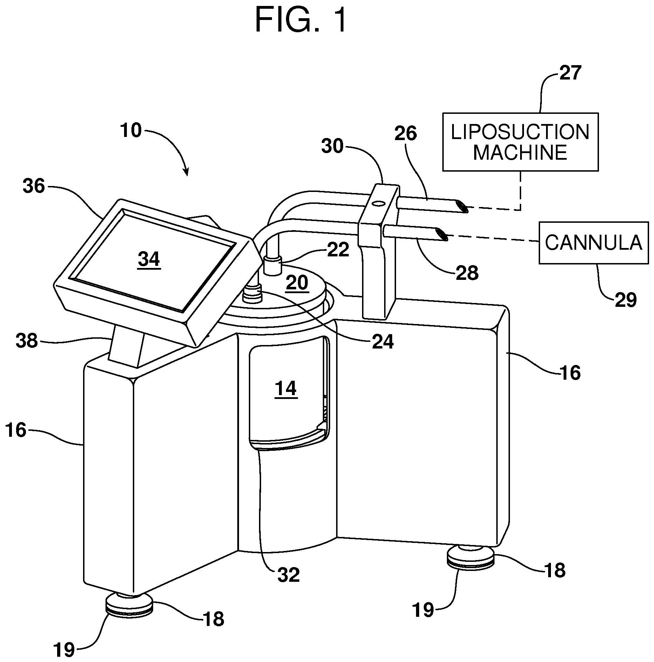

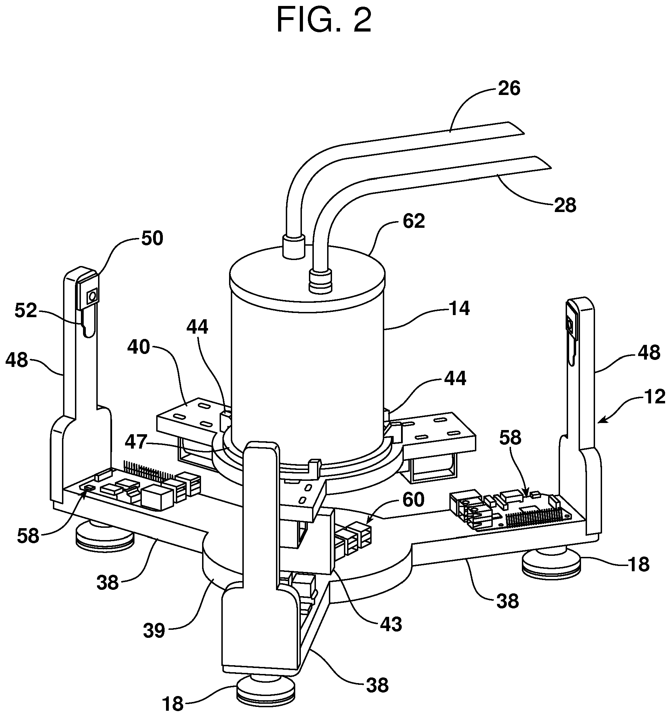

Reference is now made to Figure 1 which shows a system 10 for determining components of matter removed from a living body (e.g., a patient). The system consists of a base 12 (best shown in Figure 2) which supports a collection vessel or container 14. In the described embodiment, the container 14 is generally cylindrical in shape and centrally positioned on the base 12. The container 14 is mostly obscured and protected by a cover 16 that is attached to the base 12. The base 12 is supported by three feet 18. The feet 18 include non-skid pads 19 on bottom surfaces thereof in the described embodiment but could include wheels, casters, suction cups, etc. in alternate embodiments. In the described embodiment, the feet 18 are levelling feet in order to accommodate levelling of the base 12. A bubble level or similar mechanism may be utilized to assist in levelling the base.

The system 10 is utilized, in the described embodiment, for harvesting fat cells for grafting and the container 14 collects the matter (e.g., aspirate) removed from the patient. Alternate embodiments of the system may be utilized for component determination and/or fluid tracking in other settings including general, orthopedic, gynecologic, otolaryngology, and other surgical procedures involving blood loss. In these alternate embodiments, the system may provide an accurate volume of a blood component, for example, of the matter collected in the container for at least monitoring and possible replenishing purposes. The system may further be utilized to provide an accurate volume of a blood component of matter collected from a chest tube, wall suction containers in patient rooms, and/or urinary catheters.

In the described embodiment, a lid 20 seals the container 14 and has two ports 22, 24. An outlet port 22 for a vacuum line 26, or vacuum tube, is connected to a pump of a liposuction machine 27 and an inlet port 24 is connected by an inlet line 28, or tube, to a liposuction cannula 29 for drawing the matter out of the patient. The liposuction machine 27 and cannula 29 can be any type known in the art. The ports 22, 24 may be covered by caps until ready for use, thereby maintaining sterility of an interior of the container 14, and the container 14 is made of a translucent material in the described embodiment. Each are described in further detail below.

Both the vacuum line 26 extending between the container 14 and the liposuction machine 27 and the inlet line 28 extending between the container and the liposuction cannula 29 are immobilized by a clamp 30 extending from the base 14. The clamp 30 in the described embodiment is intended to isolate the container 14 from forces applied to one or both of the lines 26, 28. In other words, the clamp 30 ensures that neither movement nor the weight of the vacuum line 26 and/or the inlet line 28 influence a determination of a weight of the container 14. Of course, any type of device sufficient to prevent movement of the lines may be utilized whether independently supported or supported by the system.

A viewing window 32 is provided in the cover 16 for the user to visually confirm displayed output parameters and also to make observations about the matter collected in the container 14. Although not shown, a graduated scale may be provided on the container 14 to allow the user to visually quantify the matter. In an alternate embodiment, the graduated scale may be provided on the viewing window 32 itself. Different scales are provided to correspond with the various types of containers which may be used with the system. In other words, one graduated scale could apply to a container used for containment and another graduated scale could apply to a container used in the fat harvesting procedure, for example. In alternate embodiments, at least a portion of the container 14 may be transparent or translucent, or the entire container may be transparent or translucent.

A display 36, incorporating a touch screen 34 in the described embodiment, is attached to a support 38 extending from the cover 16. The display 36 is located at a convenient viewing angle and working position for the user and may be mounted for rotation in one or more axes. As will be described in more detail below, the display 36 is utilized to display information relating to the matter in the container 14 via the touch screen 34 which is effectively a user interface/display screen offering display screens including soft key buttons for receiving user inputs.

In alternate embodiments, the system 10 may include a transmitter for transmitting information including at least a weight of the matter in the container 14 and information relating to at least one aspect of the matter in the container. As will be described in more detail below, the information relating to at least one aspect of the matter in the container 14 may include a color of the matter and/or a height of the matter in the container, or other aspects. The display includes a receiver for receiving the transmitted information which is subsequently processed by a processor. In another embodiment, the system may include a transmitter for transmitting information provided by a processor, including information related to the matter in the container (e.g., a percentage of components in the matter), to a receiver positioned in the display after processing. In such a setting, information is transferred using any type of wireless technology known in the art.

In other alternate embodiments, the display may form part of a mobile device, such as, a smart phone, a personal digital assistant, or a laptop computer or tablet. In such a mobile setting, information relating to the matter in the container 14 may be transmitted from a transmitter supported by the base to the mobile device using wireless technology as is known in the art. In an alternate embodiment, such additional information may be utilized by a processor or the like supported by the base and a result or an output based at least in part on the information may be transmitted to the mobile device, or combinations of information and results transmitted. In other words, processing of the information may occur before or after transmission to the mobile device.

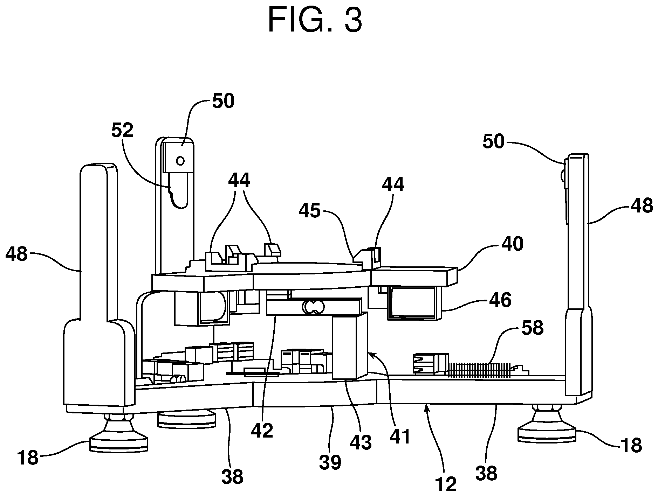

With reference to Figure 2, the base 12 is shown with the cover 16 removed generally revealing a hub and spoke type configuration. A shown, the base 12 includes three legs 38 (or spokes) extending from a hub 39. The legs 38 are supported at distal ends by feet 18. The container 14 is centrally supported by a platform 40. A device 41 is attached to the platform 40 and utilized for weighing the container 14 and the matter received in the container. As best shown in Figure 3, the device 41 in the described embodiment includes a load cell 42 although alternate embodiments may include any type of device capable of weighing the container and the matter received therein. A support 43 is attached to and extends upward from the base 12. The support 43 supports the load cell 42 and the platform 40 centrally above the hub 39.

As shown in Figures 2 and 3, the container 14 is removably connected to the platform 40 by latches 44 in the described embodiment. Figure 2 shows the container 14 connected to the platform 40 and Figure 3 shows the platform with the container removed. The latches, if used at all, may be active or passive. For example, the latches may be a resistance or a twist fitting or a powered-latch system. Even more, the latches could be replaced by any type of mechanism capable of securing an end of the container 14 adjacent the platform 40.

In the described embodiment shown in Figure 3, each latch 44 is powered by a solenoid 46 that drives the latch between first and second positions for securing and releasing the container 14. A hook portion 45 of each latch 44 extends through an aperture in the platform 40 and over a flange 47 of the container 14 in a first, secured position. To release the container 14, the solenoids 46 move the latches 44 such that the hook portion 45 does not extend over the flange 47. Securing and immobilizing the container 14 on the platform 40 in this manner ensures that an accurate weight is obtained.

As further shown in Figures 2 and 3, arms 48 extend upward from the legs 38. In the described embodiment, each arm 48 supports an instrument 50 for obtaining information relating to at least one aspect of the matter in the container 14. As suggested above, the information may include any aspect of the matter. In the described embodiment utilized in the fat cells harvesting system 10, the information obtained by the instrument includes a color of the matter and a height (volume) of the matter in the container 14.

Information concerning both the color and height of the matter in the container 14 is obtained in the described embodiment using a camera 50 as the instrument. As shown, the cameras are generally positioned adjacent the container 14. More specifically, each camera 50 is removeably supported by a support member 52 that is in turn attached to the arm 48. In the described embodiment, a camera 50 is supported by each of the arms 48 and enjoys an unobstructed view of a side of the container 14. In alternate embodiments, fewer or more cameras and/or one or more mirrors may be utilized as part of the instrument.

The use of multiple cameras allows for image averaging which increases accuracy and also allows the system 10 to obtain an average height of the matter in the event the system 10 is not level. It is worth noting here that the cover 16 prevents ambient light from interfering with the view of the container 14 as seen by the cameras 50. In alternate embodiments, supplemental lighting may be provided inside of the cover 16 to improve the images captured by the cameras for use in obtaining information relating to the matter in the container 14.

In an alternate embodiment, the instrument may be positioned adjacent a top of the container 14 (e.g., above the container). For instance, a lid 62 of the container may be transparent or clear allowing for a non-invasive sensing of the height of the matter therein. In other words, the level of the matter may be sensed using a non-invasive level sensing device as is known in the art. For example, a level sensing device may transmit a signal into the matter and determine a height based on a signal reflected from a matter-air interface.

In another possible embodiment, the instrument may include at least one optical sensor positioned adjacent the container 14. Preferably, at least one corresponding receiver would be utilized. The at least one optical sensor and receiver pairs may be utilized to obtain information relating to an aspect of the matter in the container 14. In one other embodiment, the at least one optical sensor utilizes a red beam and an infrared beam and the at least one optical receiver includes a red receiver and an infrared receiver to obtain the information.

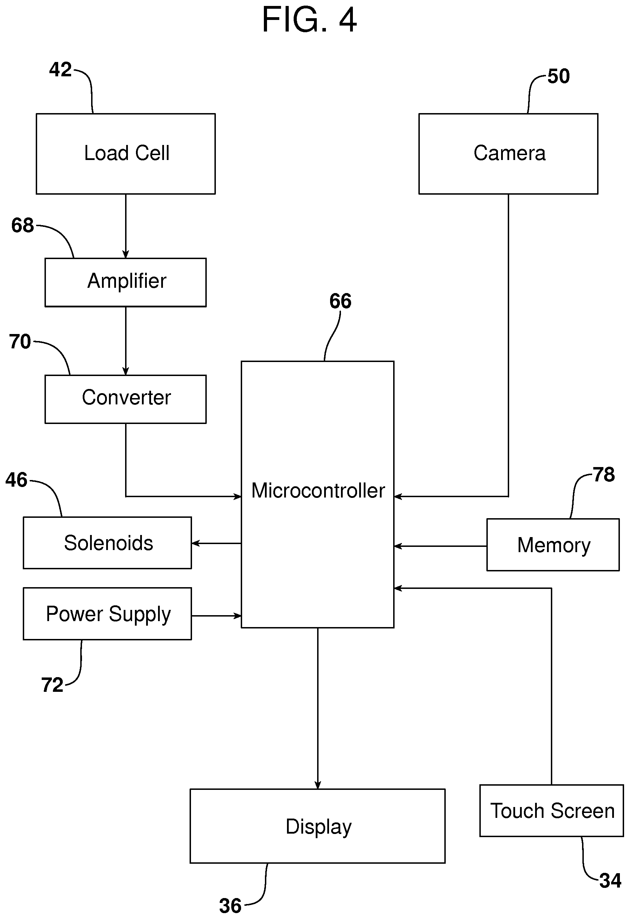

As best shown in Figure 2, the base 12 is used to support at least a portion of the electronic components of the system 10. More specifically, the base 12 supports four circuit boards in the described embodiment. A circuit board 58 is associated with each camera 50 for receiving and storing images obtained by the camera. In addition, a fourth circuit board 60 is supported by the hub 39 and receives information concerning the weight of the container 14 from the load cell 42. A power supply 72 (shown only in Figure 4), connected to line power via a power cord (not shown), is supported by the base 12 in the described embodiment. In alternate embodiments, the system 10 may utilize a battery power source and/or a battery back-up power source in the event of a power outage.

With reference to Fig 4, a general schematic of the system 10 is provided. A microcontroller 66, or processor or like device, is provided and receives inputs from each of the cameras 50 and the load cell 42. The processor 66 is programmed to determine components of the matter in the container 14 based in part on the weight of the matter determined by the load cell 42 and the information relating to the at least one aspect of the matter obtained by the cameras 50.

In the described embodiment, signals from the cameras 58 include information used in determining both the color and height of the matter in the container 14. Similarly, the load cell 42 provides an analog signal indicative of the weight of the matter in the container 14. The analog signal is amplified by amplifier 68 and converted from an analog signal to a digital signal in converter 70. From those signal inputs, the processor 66 determines a density of the matter in the container 14. Understanding that the density of fat cells is known to be approximately 0.9 g/cm³, water is 1.0 g/cm³, and blood is 1.1 g/cm³, the processor 66 can determine a quantity of the matter which is a fat cells component and a quantity which is a fluid component.

Even more, the processor 66 can determine an amount of a blood component within the fluid component based in part on information relating to the color of the matter. This information is used in the aforementioned alternate embodiments of the system utilized for fluid tracking and component determination in other settings involving blood loss. In these alternate embodiments, the system 10 would provide an accurate volume of a blood component of the matter collected in the container 14 for at least monitoring and possible replenishing purposes. The system may further be utilized to provide an accurate volume of a blood component of matter collected from a chest tube, wall suction containers in patient rooms, and/or urinary catheters.

As noted above, the processor 66 is programmed to analyze the signals from the cameras 50, including the at least one image, to determine the height of the matter in the container 14. The information obtained may further include information relating to a height of the fat cells component in the container. In an alternate embodiment, the system 10 may include a digital pattern recognition analysis system to analyze the at least one image from the cameras 50 to determine the height of the matter in the container 14.

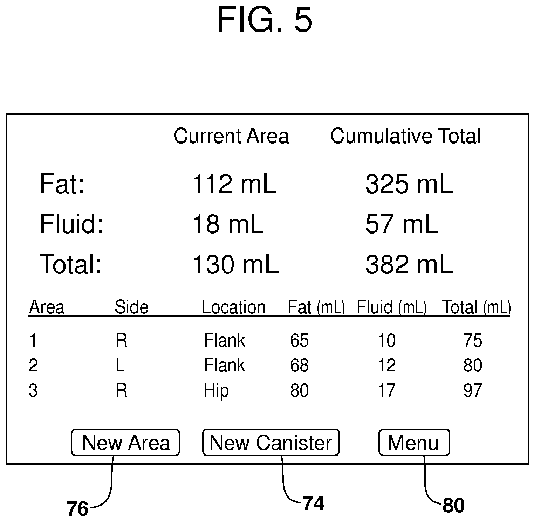

As described above, the display 36 incorporates a touch screen 34 which displays information relating to the matter in the container 14. As noted, the touch screen 34 is effectively a user interface/display screen offering display screens including soft key or fixed buttons for receiving user inputs. In other words, the touch screen 34 accepts user inputs and displays information output from the system 10. Figure 5 illustrates an exemplary display screen.

Upon startup, the system 10 will automatically tare the weight of the container 14 and stabilized lines 26, 28. The touch screen 34 displays a soft key 74 labelled "New Container" to indicate that a new container has been secured to the platform. When depressed, the "New Container" soft key 74 signals the processor 66 to tare the weight as described above. Throughout operation, the display 36 via touch screen 34 provides real time measurements of fat volume, fluid volume, and total volume. For example, the "Fat" volume in "Area 1" is shown as 65 ml and the "Fluid" volume as 10 ml. As shown, the volumes are displayed in milliliters although other units of measure may be utilized.

When the user/surgeon has completed suctioning in a first area, a "New Area" soft key 76 may be depressed indicating that further suctioning will occur in a different, second area of the living body or patient. Once the soft key 76 is depressed, the user is directed to a secondary menu screen to input parameters relating to the new area. For example, the user may select a side of the patient and a location from a listing of such items or may key in such information using a soft key keyboard for example. The data from each completed area is retained by the system 10 in a memory 78 and displayed in a scroll menu below a larger "Current Area" and "Cumulative Total" headings above the actual readings. As shown, an "Area" "1" may be a "Flank" on the right side ("R") of the patient or an "Area" "2" may be a "Flank" on a left side ("L") of the patient.

In alternate embodiments, the soft key 76 which acts as a switch within the touch screen 34 may take the form of a physical switch (e.g., a momentary switch or a foot switch). Such a physical switch could be mounted on the cannula or positioned on the operating room floor in order to provide convenient access without interrupting an ongoing procedure.

If the container 14 reaches maximum capacity, i.e., the container has reached a predetermined level or is completely full, then pressing the "New Container" soft key 74 will indicate a desire to remove and replace the original container with a new container. Accordingly, the processor 66 will activate the solenoids 46 to move the latches 44 thereby releasing the flange 47 and the container 14. After placement of the new container, pressing the "New Container" soft key 74 will cause the latches 44 to secure the new container 14' and the system 10 will again tare while providing a continuation of data acquisition in the current area. A "Menu" soft key 80 allows the user to reset the system 10 for a new procedure or patient, or to change display parameters, units, etc.

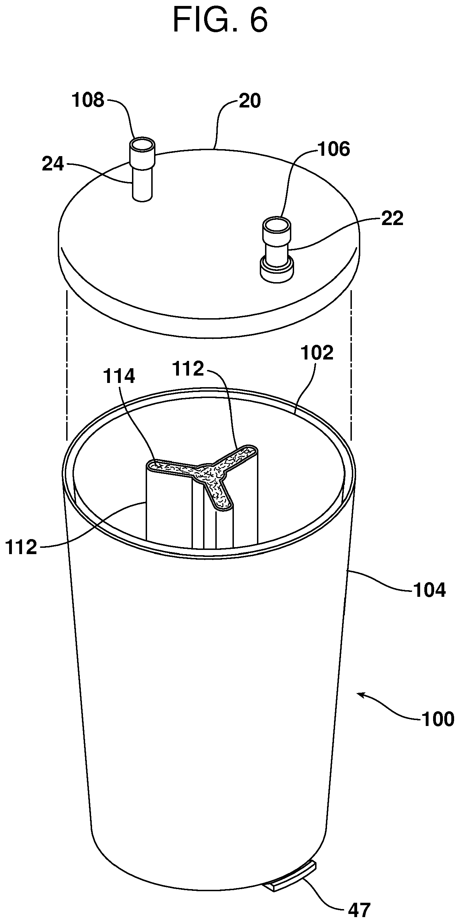

As described above, the system 10 is utilized, in the described embodiment, for harvesting fat cells for grafting and the container 14 collects the matter (e.g., aspirate) removed from the patient. Ideally in such a procedure, the system 10 would efficiently separate fat cells from the fluid, or slurry, within the matter removed from the patient, i.e., the liposuction aspirate, while minimizing mechanical trauma to the fat cells. Even more, the system 10 would retain the separated fat cells in a sterile container that may be transferred onto a surgical field for grafting. To provide these capabilities, a container 100, in the described embodiment, includes two nested containers, i.e., inner and outer containers 102, 104 as shown in Figure 6.

For the alternate embodiments of the system utilized for fluid tracking and component determination in other settings including general, orthopedic, gynecologic, otolaryngology, and other surgical procedures involving blood loss, the use of nested containers is not required and the container 14 described above would be sufficient. For the described embodiment, however, the container 100 includes inner and outer containers 102, 104 in order to separate fat cells from the fluid within the matter removed from the patient. The elements of the container 14 described above which remain unchanged in the container 100 (e.g., lid 20) will maintain the same reference numerals.

A lid 20, shown separated from outer container 104 in Figure 6, seals both inner and outer containers 102, 104. As described above, the lid 20 has two ports 22, 24. A first port 22 for connection to a vacuum line 26 connected to the liposuction machine 27 and a second port 24 connected a cannula 29 for drawing the aspirate out of the patient. As indicated above, the ports 22, 24 are covered by caps 106, 108 until ready for use, thereby maintaining sterility. Both inner and outer containers 102, 104 are made of a clear material and may include graduations to allow the user to determine the quantity of aspirate or its components as described above. A flange 47 extends from outer container 104 for securing the container 100 to the platform 40 as described above. The flange 47 may extend entirely around the container or, in alternate embodiments, may include several flange segments as shown in Figure 6.

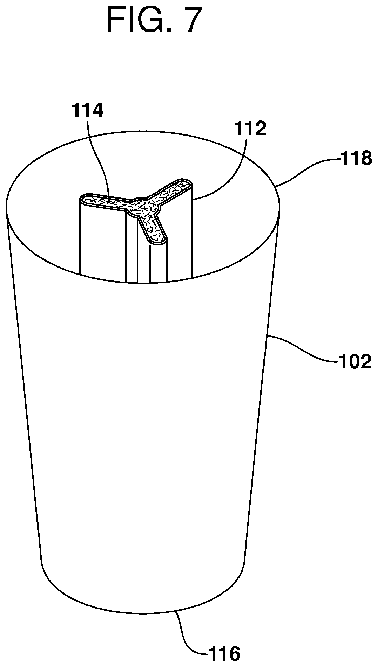

As best shown in Figure 7, an insert 112 is centrally positioned within the inner container 102 and extends from a bottom 116 to a top 118 of the inner container 102. The insert 112 contains an absorbent material 114 suitable for absorbing liquid. In the described embodiment, a superabsorbent material, such as sodium polyacryate, with a high absorbent capacity is utilized. Superabsorbent polymers and hydrogels may likewise be utilized and are commonly utilized in disposable diapers for their high fluid binding ability. In one alternate embodiment, the insert 112 may consist of multiple packets of superabsorbent material or may utilize such multiple packets.

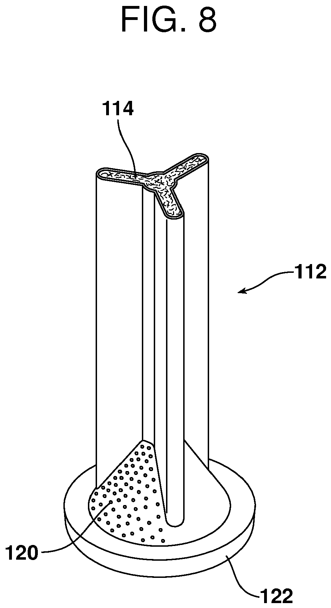

Even more, the insert 112 is designed, as depicted in Figure 8, to maximize a surface area in contact with the matter deposited in the inner container 102 during the liposuction process. Multiple small holes 120, or a fine mesh filter, a paper filter, a one-way fabric or the like positioned over apertures, in the insert 112 allow the fluid component of the matter to flow into the insert and contact the superabsorbent material 114. As the matter flows into the container 100, the fluid component is absorbed into and trapped by the superabsorbent material 114. Although not shown, the small holes 120 exist on each and every surface of the insert 112 in the described embodiment. Alternate embodiments, however, may limit the surfaces on which the small holes 120 are positioned or the size and/or number of small holes in general.

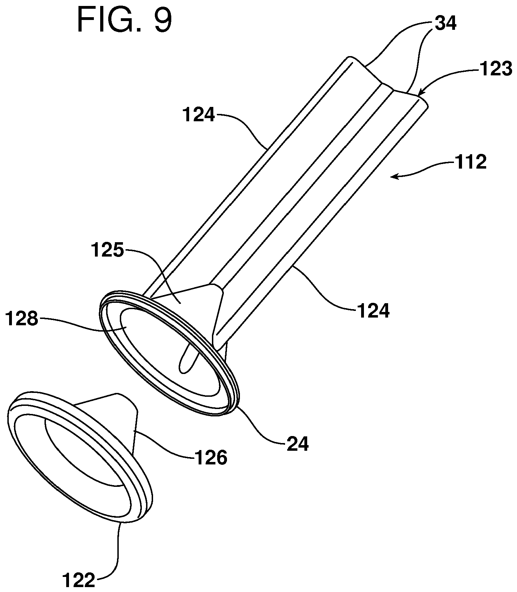

As shown in Figure 9, the described insert 112 includes a separable base 122 which extends across a substantially full diameter of the bottom 116 of the inner container 102 and acts as a receiver. The insert 112 also has a vertical upward extension 123 of a tri-foil configuration, i.e., the insert includes a plurality of fins 124 extending outward from a central, lengthwise axis, to maximize the surface area of the insert while minimizing a maximum distance between the superabsorbent material 116 and the matter. The vertical upward extension 123 also includes a lower cone-shaped, or truncated cone-shaped, portion 125 configured to receive a like cone-shaped or truncated cone-shaped portion 126 forming a part of and extending upward from the base 122. The superabsorbent material 114 may be positioned between the cone-shaped portions 125, 126 and throughout the upward extension 123 and fins 124. Other embodiments may include more or less superabsorbent material 114 and more or fewer fins 124.

In one alternate embodiment, the insert 112 could be integrally molded with the inner container 102 rather than exist as a separate component. Alternatively, the insert 112 could include a handle or like mechanism to facilitate removal of the insert from the inner container 102. A further alternate embodiment could have the superabsorbent material 114 reside within the outer container 104 and be brought into direct or indirect contact with the inner container 102 (e.g., via a central column having a large surface area). With these configurations, when the user removes the inner container 102, the saturated superabsorbent would already have been removed or would remain with the outer container and only the fat cells to be used for grafting would remain in the inner container. A handle (not shown) could be provided on the inner container 102 to assist with its removal from the outer container 104.

In the described embodiment, the inner and outer containers 102, 104, the lid 20 and the insert 112 with its superabsorbent material 114, are supplied to the user sterilely. Outer surfaces of the outer container 104 and the lid 20 will become contaminated as they are handled non-sterilely and may be attached to the non-sterile liposuction machine. However, interior surfaces of the outer container 104 and lid 20 remain sterile along with the inner container 102. The interior of tubing 28 connecting the cannula 29 to the inlet port 24 is also sterile. To maintain a sterile path for matter or aspirate flow, the inlet port 24 is covered by a sterile cap 108 (shown in Figure 6) which is removed immediately prior to attachment of the tubing. Similarly, the user removes a second cap 106 and attaches the vacuum line 26 leading to the liposuction machine 27.

In accordance with a method of determining components of matter removed from a living body, a patient, matter removed from the living body is directed into a container 14. In the described embodiment, a vacuum is applied to the cannula 29 of the system 10 by a liposuction machine 27 and the matter, or fat aspirate in this instance, is directed into the container 14. The matter and the container 14 are weighed and a weight of the matter is determined by a processor 66 which monitors an output of a load cell 42 in the described embodiment. As described above, a tare weight of the container 14 is determined using the load cell 42 before the matter is directed into the container and utilized by the processor 66. The load cell 42 is then utilized to ascertain a combined weight.

Information relating to at least one aspect of the matter in the container 14 is also obtained. In the described embodiment, the information is obtained using a plurality of cameras 50. As described above, the cameras 50 are mounted on arms 48 and positioned adjacent the container 14 for capturing images of the matter in the container. Based in part on the weight of the matter and the information relating to at least one aspect of the matter, the processor 66 is programmed to determine the components of the matter in the container 14. In the described embodiment, the components include a fat cells component and a fluid component.

In alternate embodiments, the system 10 may be utilized for component determination and/or fluid tracking in other settings involving blood loss. These may include general, orthopedic, gynecologic, otolaryngology, and other surgical procedures for example. In conjunction with the needs associated with these alternate procedures, the system 10 may provide an accurate volume of a blood component, for example, of the matter collected in the container 14 rather than a fat cells component. This may be done for at least monitoring and possibly replenishing purposes. In such alternate embodiments, the information relating to at least one aspect of the matter in the container 14 includes information relating to a color of the matter. More specifically, the cameras 50 or other instruments are used to determine the color of the matter which is subsequently used in determining the amount of the blood component of the matter. This is determined in part on the weight and the color of the matter in the container 14.

The information obtained using the plurality of cameras 50, or other instruments, may also relate to a height of the matter in the container 14. In the described embodiment, the height of the matter in the container 14 is obtained by analyzing the image(s) of the matter. Information relating to the height of the matter in the container 14 is used to determine the components of the matter.

In an alternate embodiment described above, the fluid component is separated from the fat cells component of the matter in the container 100. The fluid component is drawn away from the fat cells component through a filter using an absorbent material. The filter may include a plurality of holes 120 in an insert 112 of the container 100 or filter paper or the like as described above. In this embodiment, the plurality of cameras 50, or other instruments, obtain information relating to a height of the fat cells component of the matter in the container 14. Similarly, the height of the fat cells component is obtained by analyzing the image(s) of the matter. In this instance, however, the components are determined based in part on the weight of the matter and the height of the fat cells component.

In summary, numerous benefits result from providing a system for determining components of matter removed from a living body. For instance, blood loss during procedures may be monitored and possibly replenished. Even more, fat cells harvested from a patient may be quantitatively monitored for purposes of ensuring equivalent removal from paired body areas or to ensure a sufficient amount of fat cells for grafting purposes. In the fat grafting embodiment, the system allows the fat cells to be rapidly separated from fluid in a liposuction aspirate mixture and the absorbent materials utilized within the separating process allows for an atraumatic separation.. In this manner, the separated fat cells should be ready for re-injection with minimal delay. Even more, the fat cells may be separated and re-injected without leaving the sterile operating environment.

The foregoing has been presented for purposes of illustration and description. It is not intended to be exhaustive or to limit the embodiments to the precise form disclosed. Obvious modifications and variations are possible in light of the above teachings. For example, alternate embodiments may utilize the superabsorbent materials within the container in non-fat cell harvesting procedures in order to increase the accuracy of the component determining system. All such modifications and variations are within the scope of the appended claims when interpreted in accordance with the breadth to which they are fairly, legally and equitably entitled.

## Claims

1. A system for determining components of matter removed from a living body, comprising:
a container (14) for receiving the matter removed from the living body via an inlet tube (28);
a load cell (42) for weighing said container and the matter received therein;
at least one optical sensor positioned adjacent said container and at least one corresponding receiver (50) for obtaining information relating to a colour of the matter in said container; and
a processor (66) programmed to determine the components of the matter in said container based in part on a weight of the matter determined by said load cell and the information relating to a colour of the matter obtained by said at least one optical sensor and corresponding receiver (50).

2. The system for determining components of matter removed from a living body of claim 1, further comprising a base (12) supporting said container (14), said load cell (42), and said at least one optical sensor and corresponding receiver (50).

3. The system for determining components of matter removed from a living body of claim 1, wherein said at least one optical sensor and corresponding receiver (50) obtains information relating to a height of the matter in said container (14).

4. The system for determining components of matter removed from a living body of claim 1, wherein said at least one optical sensor and corresponding receiver (50) obtains information relating to a color of the matter in said container.

5. The system for determining components of matter removed from a living body of claim 4, wherein said processor (66) is programmed to determine an amount of a blood component of the matter based in part on the weight of the matter determined by said load cell (42) and the color of the matter in said container (14).

6. The system for determining components of matter removed from a living body of claim 1, further comprising a memory (78) associated with said processor (66) for storing at least the weight of the matter determined by said load cell and the information relating to the colour of the matter obtained by said at least one optical sensor and at least one corresponding receiver (50).

7. The system for determining components of matter removed from a living body of claim 6, further comprising a switch (76) for indicating to said processor (66) a transition from one area of the living body from which the matter was removed to another area of the living body from which additional matter is to be removed.

8. The system for determining components of matter removed from a living body of claim 1, further comprising a pump (27) connected to said container (14) via a vacuum tube (26), said pump creating a vacuum within the system for suction the matter from the living body, and a cannula (29) connected to said container (14) via said inlet tube (28), said cannula configured to be partially inserted into the living body for suctioning the matter from the living body.

9. The system for determining components of matter removed from a living body of claim 1, further comprising an insert (112) positioned within said container (14) and containing an absorbent material (114), wherein the matter in use includes a fat cells component and a fluid component, and said insert is porous allowing the fluid component to enter said insert for absorption by said absorbent material.

10. The system for determining components of matter removed from a living body of claim 9, further comprising an inner container (102) positioned within said container (14) for receiving the matter from the patient, said inner container supporting said insert (112).

11. The system for determining components of matter removed from a living body of claim 10, wherein said processor (66) is programmed to determine whether said container (14) includes said inner container (102).

12. The system for determining components of matter removed from a living body of claim 1, wherein said at least one optical sensor utilizes a red beam and an infrared beam and said at least one corresponding receiver utilizes a red receiver and an infrared receiver.

## Patentansprüche

1. System zum Bestimmen von Bestandteilen der aus einem lebenden Körper entnommenen Materie, umfassend:
einen Behälter (14) zum Aufnehmen der aus dem lebenden Körper entnommenen Materie über ein Einlassrohr (28);
eine Wägezelle (42) zum Wiegen des genannten Behälters und der darin enthaltenen Materie;
mindestens einen optischer Sensor, der neben dem Behälter angeordnet ist, und mindestens einen entsprechenden Empfänger (50) zum Erhalten von Informationen bezüglich einer Farbe der Materie in dem Behälter; und
einen Prozessor (66), der so programmiert ist, dass er die Bestandteile der Materie in dem Behälter teilweise auf der Grundlage eines Gewichtes der Materie, das durch die Wägezelle bestimmt wird, und der Information bezüglich einer Farbe der Materie, die durch den mindestens einen optischen Sensor und entsprechenden Empfänger (50) erhalten wird, bestimmt.

2. System zum Bestimmen von Bestandteilen der aus einem lebenden Körper entnommenen Materie nach Anspruch 1, ferner umfassend eine Basis (12), die den Behälter (14), die Wägezelle (42) und den mindestens einen optischen Sensor und den entsprechenden Empfänger (50) trägt.

3. System zum Bestimmen von Bestandteilen der aus einem lebenden Körper entnommenen Materie nach Anspruch 1, wobei der mindestens eine optische Sensor und der entsprechende Empfänger (50) Informationen bezüglich einer Höhe der Materie in dem Behälter (14) erhalten.

4. System zum Bestimmen von Bestandteilen der aus einem lebenden Körper entnommenen Materie nach Anspruch 1, wobei der mindestens eine optische Sensor und der entsprechende Empfänger (50) Informationen bezüglich einer Farbe der Materie in dem Behälter erhalten.

5. System zum Bestimmen von Bestandteilen der aus einem lebenden Körper entnommenen Materie nach Anspruch 4, wobei der Prozessor (66) programmiert ist, um eine Menge eines Blutbestandteils der Materie zu bestimmen, die teilweise auf dem Gewicht der Materie, das durch die Wägezelle (42) bestimmt wird, und der Farbe der Materie in dem Behälter (14) basiert.

6. System zum Bestimmen von Bestandteilen der aus einem lebenden Körper entnommenen Materie nach Anspruch 1, ferner umfassend einen Speicher (78), der mit dem Prozessor (66) verbunden ist, um mindestens das Gewicht der durch die Wägezelle bestimmten Materie und die Information bezüglich der Farbe der Materie, die durch den mindestens einen optischen Sensor und den mindestens einen entsprechenden Empfänger (50) erhalten wurde, zu speichern.

7. System zum Bestimmen von Bestandteilen der aus einem lebenden Körper entnommenen Materie nach Anspruch 6, ferner umfassend einen Schalter (76), um dem Prozessor (66) einen Übergang von einem Bereich des lebenden Körpers, aus dem die Materie entnommen wurde, zu einem anderen Bereich des lebenden Körpers, aus dem zusätzliche Materie zu entnehmen ist, anzuzeigen.

8. System zum Bestimmen von Bestandteilen der aus einem lebenden Körper entnommenen Materie nach Anspruch 1, ferner umfassend eine Pumpe (27), die über ein Vakuumrohr (26) mit dem Behälter (14) verbunden ist, wobei die Pumpe innerhalb des Systems ein Vakuum erzeugt, um die Materie aus dem lebenden Körper abzusaugen, und eine Kanüle (29), die über das Einlassrohr (28) mit dem Behälter (14) verbunden ist, wobei die Kanüle so konfiguriert ist, dass sie teilweise in den lebenden Körper eingeführt werden kann, um die Materie aus dem lebenden Körper abzusaugen.

9. System zum Bestimmen von Bestandteilen der aus einem lebenden Körper entnommenen Materie nach Anspruch 1, ferner umfassend einen Einsatz (112), der in dem Behälter (14) angeordnet ist und ein absorbierendes Material (114) enthält, wobei die verwendete Materie einen Fettzellenbestandteil und einen Fluidbestandteil enthält und wobei der Einsatz porös ist, so dass der Fluidbestandteil zur Absorption durch das absorbierende Material in den Einsatz eintreten kann.

10. System zum Bestimmen von Bestandteilen der aus einem lebenden Körper entnommenen Materie nach Anspruch 9, ferner umfassend einen innerhalb des Behälters (14) angeordneten Innenbehälter (102) zur Aufnahme der Materie vom Patienten, wobei der Innenbehälter den Einsatz (112) trägt.

11. System zum Bestimmen von Bestandteilen der aus einem lebenden Körper entnommenen Materie nach Anspruch 10, wobei der Prozessor (66) programmiert ist, um zu bestimmen, ob der Behälter (14) den inneren Behälter (102) enthält.

12. System zum Bestimmen von Bestandteilen der aus einem lebenden Körper entnommenen Materie nach Anspruch 1, wobei der mindestens eine optische Sensor einen roten Strahl und einen Infrarotstrahl verwendet und wobei der mindestens eine entsprechende Empfänger einen roten Empfänger und einen Infrarotempfänger verwendet.

## Revendications

1. Système de détermination de composants de matière éliminée d'un corps vivant, comprenant :
un récipient (14) destiné à recevoir la matière éliminée du corps vivant par l'intermédiaire d'un tube d'entrée (28) ;
une cellule de charge (42) destinée à peser ledit récipient et la matière contenue dans ce dernier ;
au moins un capteur optique positionné de manière adjacente audit récipient et au moins un récepteur correspondant (50) destiné à obtenir des informations relatives à une couleur de la matière dans ledit récipient ; et
un processeur (66) programmé pour déterminer les composants de la matière dans ledit récipient sur la base, en partie, d'un poids de la matière déterminé par ladite cellule de charge et des informations relatives à une couleur de la matière obtenues par ledit au moins un capteur optique et récepteur correspondant (50).

2. Système de détermination de composants de matière éliminée d'un corps vivant selon la revendication 1, comprenant en outre une base (12) soutenant ledit récipient (14), ladite cellule de charge (42), et ledit au moins un capteur optique et récepteur correspondant (50).

3. Système de détermination de composants de matière éliminée d'un corps vivant selon la revendication 1, dans lequel ledit au moins un capteur optique et récepteur correspondant (50) obtient des informations relatives à une hauteur de la matière dans ledit récipient (14).

4. Système de détermination de composants de matière éliminée d'un corps vivant selon la revendication 1, dans lequel ledit au moins un capteur optique et récepteur correspondant (50) obtient des informations relatives à une couleur de la matière dans ledit récipient.

5. Système de détermination de composants de matière éliminée d'un corps vivant selon la revendication 4, dans lequel ledit processeur (66) est programmé pour déterminer une quantité d'un composant sanguin de la matière sur la base, en partie, du poids de la matière déterminé par ladite cellule de charge (42) et la couleur de la matière dans ledit récipient (14).

6. Système de détermination de composants de matière éliminée d'un corps vivant selon la revendication 1, comprenant en outre une mémoire (78) associée audit processeur (66) pour stocker au moins le poids de la matière déterminé par ladite cellule de charge et les informations concernant la couleur de la matière obtenue par ledit au moins un capteur optique et au moins un récepteur correspondant (50).

7. Système de détermination de composants de matière éliminée d'un corps vivant selon la revendication 6, comprenant en outre un commutateur (76) pour indiquer audit processeur (66) une transition d'une zone du corps vivant dont la matière a été éliminée vers une autre zone du corps vivant à partir de laquelle des matières supplémentaires doivent être éliminées.

8. Système de détermination de composants de matière éliminée d'un corps vivant selon la revendication 1, comprenant en outre une pompe (27) reliée audit récipient (14) par l'intermédiaire d'un tube à vide (26), ladite pompe créant un vide à l'intérieur du système pour aspirer la matière du corps vivant, et une canule (29) reliée audit récipient (14) par l'intermédiaire dudit tube d'entrée (28), ladite canule étant configurée pour être partiellement insérée dans le corps vivant afin d'aspirer la matière du corps vivant.

9. Système de détermination de composants de matière éliminée d'un corps vivant selon la revendication 1, comprenant en outre un insert (112) positionné à l'intérieur dudit récipient (14) et contenant un matériau absorbant (114), dans lequel la matière utilisée comprend un composant de cellules adipeuses et un composant fluide, et ledit insert est poreux permettant au composant fluide d'entrer dans ledit insert pour être absorbé par ledit matériau absorbant.

10. Système de détermination de composants de matière éliminée d'un corps vivant selon la revendication 9, comprenant en outre un récipient intérieur (102) positionné à l'intérieur dudit récipient (14) pour recevoir la matière du patient, ledit récipient intérieur soutenant ledit insert (112).

11. Système de détermination de composants de matière éliminée d'un corps vivant selon la revendication 10, dans lequel ledit processeur (66) est programmé pour déterminer le fait que ledit récipient (14) comprend ledit récipient intérieur (102).

12. Système de détermination de composants de matière éliminée d'un corps vivant selon la revendication 1, dans lequel ledit au moins un capteur optique utilise un faisceau rouge et un faisceau infrarouge et ledit au moins un récepteur correspondant utilise un récepteur rouge et un récepteur infrarouge.
